# EUROPÄISCHE PATENTANMELDUNG

(11) **EP 0 832 901 A1**
(43) Veröffentlichungstag der Anmeldung: **01.04.1998**
(21) Anmeldenummer: 97116260.7
(22) Anmeldetag: 18.09.1997
(51) Int. Cl.: C07K 16/10, C07K 19/00, A61K 47/48, A61K 39/42, C12N 5/20, C07K 14/18

(54) **Antikörper gegen Hepatitis G-Virus und deren Verwendung zum diagnostischen Nachweis von HGV sowie als Therapeutikum**

(30) Priorität: 18.09.1996 DE 19638133; 22.10.1996 DE 19643650
(71) Anmelder: BOEHRINGER MANNHEIM GMBH, 68305 Mannheim (DE)
(72) Erfinder: Schmolke, Susanne, Dr., 82377 Penzberg (DE); Tacke, Michael, Dr., 82377 Penzberg (DE); Hübner-Parajsz, Christa, Dr., 82327 Tutzing (DE); Engel, Alfred, Dr., 82377 Penzberg (DE); Ofenloch-Hähnle, Beatus, Dr., 82398 Polling (DE)
(74) Vertreter: Weiss, Wolfgang, Dipl.-Chem. Dr.

(57) **Zusammenfassung**

Die vorliegende Erfindung betrifft einen Antikörper gegen ein Hepatitis-G-Virus-Antigen sowie ein Fragment dieses Antikörpers. Die Erfindung betrifft außerdem Hybridomzellinien sowie ein Konjugat, welches diesen Antikörper oder das Antikörperfragment gekoppelt, an ein biologisches Molekül enthält.

Schließlich betrifft die vorliegende Erfindung die Verwendung des Antikörpers zum diagnostischen Nachweis von Hepatitis G-Virus.

SEQUENZPROTOKOLL

(1) ALLGEMEINE ANGABEN:

(i) ANMELDER:
(A) NAME: Boehringer Mannheim GmbH
(B) STRASSE: Sandhofer Str. 112-132
(C) ORT: Mannheim
(E) LAND: DE
(F) POSTLEITZAHL: 68305

(ii) BEZEICHNUNG DER ERFINDUNG: Antikoerper gegen Hepatitis G-Virus und
deren Verwendung zum diagnostischen Nachweis von HGV sowie
als Therapeutikum

(iii) ANZAHL DER SEQUENZEN: 2

(iv) COMPUTER-LESBARE FASSUNG:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:
(A) LÄNGE: 1302 Basenpaare
(B) ART: Nucleotid
(C) STRANGFORM: beides
(D) TOPOLOGIE: linear

(vi) URSPRÜNLICHE HERKUNFT:
(A) ORGANISMUS: Hepatitis G Virus

(vii) UNMITTELBARE HERKUNFT:
(B) CLON(E): E1TM

(ix) MERKMAL:
(A) NAME/SCHLÜSSEL: CDS
(B) LAGE:13..1290

(xi) SEQUENZBESCHREIBUNG: SEQ ID NO: 1:

GAATTCACCA CC ATG GGG GTG CAC GAA TGT CCT GCC TGG CTG TGG CTT 48
Met Gly Val His Glu Cys Pro Ala Trp Leu Trp Leu
1               5                   10

CTC CTG TCC CTG CTG TCG CTC CCT CTG GGC CTC CCA GTC CTG GGC GAC 96
Leu Leu Ser Leu Leu Ser Leu Pro Leu Gly Leu Pro Val Leu Gly Asp
        15                  20                  25

TAC AAG GAC GAT GAC GAT AAG GCG GCC GCG ATG TCG CAA GGC GCC CCT 144
Tyr Lys Asp Asp Asp Asp Lys Ala Ala Ala Met Ser Gln Gly Ala Pro
    30                  35                  40

GCC TCC GTT TTG GGG TCA CGC CCC TTT GAC TAC GGG TTG ACT TGG CAG 192
Ala Ser Val Leu Gly Ser Arg Pro Phe Asp Tyr Gly Leu Thr Trp Gln
45                  50                  55                  60

ACC TGC TCT TGC AGG GCC AAC GGT TCG CGT TTT TCG ACT GGG GAG AAG 240

Thr Cys Ser Cys Arg Ala Asn Gly Ser Arg Phe Ser Thr Gly Glu Lys
                65                  70                  75

GTG TGG GAC CGT GGG AAC GTT ACG CTT CAG TGT GAC TGC CCT AAC GGC      288
Val Trp Asp Arg Gly Asn Val Thr Leu Gln Cys Asp Cys Pro Asn Gly
            80                  85                  90

CCC TGG GTG TGG TTG CCA GCC TTT TGC CAA GCA ATC GGC TGG GGT GAC      336
Pro Trp Val Trp Leu Pro Ala Phe Cys Gln Ala Ile Gly Trp Gly Asp
        95                  100                 105

CCC ATC ACT TAT TGG AGC CAC GGG CAA AAT CAG TGG CCC CTT TCA TGC      384
Pro Ile Thr Tyr Trp Ser His Gly Gln Asn Gln Trp Pro Leu Ser Cys
    110                 115                 120

CCC CAG TAT GTC TAT GGG TCT GCT ACA GTC ACT TGC GTG TGG GGT TCC      432
Pro Gln Tyr Val Tyr Gly Ser Ala Thr Val Thr Cys Val Trp Gly Ser
125                 130                 135                 140

GCT TCT TGG TAT GCC TCC ACC AGT GGT CGC GAC TCG AAG ATA GAT GTG      480
Ala Ser Trp Tyr Ala Ser Thr Ser Gly Arg Asp Ser Lys Ile Asp Val
                145                 150                 155

TGG AGT TTA GTG CCA GTT GGC TCT GCC ACC TGC ACC ATA GCC GCA CTT      528
Trp Ser Leu Val Pro Val Gly Ser Ala Thr Cys Thr Ile Ala Ala Leu
            160                 165                 170

GGA TCA TCG GAT CGC GAC ACG GTG CCT GGG CTC TCC GAG TGG GGA ATC      576
Gly Ser Ser Asp Arg Asp Thr Val Pro Gly Leu Ser Glu Trp Gly Ile
        175                 180                 185

CCG TGC GTG ACG TGT GTT CTG GAC CGT CGG CCT GCT TCA TGC GGC ACC      624
Pro Cys Val Thr Cys Val Leu Asp Arg Arg Pro Ala Ser Cys Gly Thr
    190                 195                 200

TGT GTG AGG GAC TGC TGG CCC GAG ACC GGG TCG GTT AGG TTC CCA TTC      672
Cys Val Arg Asp Cys Trp Pro Glu Thr Gly Ser Val Arg Phe Pro Phe
205                 210                 215                 220

CAT CGG TGC GGC GTG GGG CCT CGG CTG ACA AAG GAC TTG GAA GCT GTG      720
His Arg Cys Gly Val Gly Pro Arg Leu Thr Lys Asp Leu Glu Ala Val
                225                 230                 235

CCC TTC GTC AAT AGG ACA ACT CCC TTC ACC ATT AGG GGG CCC CTG GGC      768
Pro Phe Val Asn Arg Thr Thr Pro Phe Thr Ile Arg Gly Pro Leu Gly
            240                 245                 250

AAC CAG GGC CGA GGC AAC CCG GTG CGG TCG CCC TTG GGT TTT GGG TCC      816
Asn Gln Gly Arg Gly Asn Pro Val Arg Ser Pro Leu Gly Phe Gly Ser
        255                 260                 265

TAC GCC ATG ACC AGG ATC CGA GAT ACC CTA CAT CTG GTG GAG TGT CCC      864
Tyr Ala Met Thr Arg Ile Arg Asp Thr Leu His Leu Val Glu Cys Pro
    270                 275                 280

ACA CCA GCC ATC GAG CCT CCC ACC GGG ACG TTT GGG TTC TTC CCC GGG      912
Thr Pro Ala Ile Glu Pro Pro Thr Gly Thr Phe Gly Phe Phe Pro Gly
285                 290                 295                 300

ACG CCG CCT CTC AAC AAC TGC ATG CTC TTG GGC ACG GAA GTG TCC GAG      960
Thr Pro Pro Leu Asn Asn Cys Met Leu Leu Gly Thr Glu Val Ser Glu
                305                 310                 315

GCA CTT GGG GGG GCT GGC CTC ACG GGG GGG TTC TAT GAA CCC CTG GTG     1008

Ala Leu Gly Gly Ala Gly Leu Thr Gly Gly Phe Tyr Glu Pro Leu Val
            320                 325                 330

CGC AGG TGT TCG GAG CTG ATG GGA AGC CGA AAT CCG GTT TGT CCG GGG        1056
Arg Arg Cys Ser Glu Leu Met Gly Ser Arg Asn Pro Val Cys Pro Gly
        335                 340                 345

TTT GCA TGG CTC TCT TCG GGC AGG CCT GAT GGG TTT ATA CAT GTC CAG        1104
Phe Ala Trp Leu Ser Ser Gly Arg Pro Asp Gly Phe Ile His Val Gln
    350                 355                 360

GGT CAC TTG CAG GAG GTG GAT GCA GGC AAC TTC ATC CCG CCC CCG CGC        1152
Gly His Leu Gln Glu Val Asp Ala Gly Asn Phe Ile Pro Pro Pro Arg
365                 370                 375                 380

TGG TTG CTC TTG GAC TTT GTA TTT GTC CTG TTA TAC CTG ATG AAG CTG        1200
Trp Leu Leu Leu Asp Phe Val Phe Val Leu Leu Tyr Leu Met Lys Leu
                385                 390                 395

GCT GAG GCA CGG TTG GTC CCG CTG ATC TTG CTG CTG CTA TGG TGG TGG        1248
Ala Glu Ala Arg Leu Val Pro Leu Ile Leu Leu Leu Leu Trp Trp Trp
            400                 405                 410

GTG AAC CAG CTG GCA GTC CTA GGG CTG CCG GCT GTG GAA GCC                1290
Val Asn Gln Leu Ala Val Leu Gly Leu Pro Ala Val Glu Ala
        415                 420                 425

TAATAGTCTA GA                                                          1302

(2) ANGABEN ZU SEQ ID NO: 2:

    (i) SEQUENZKENNZEICHEN:
        (A) LÄNGE: 426 Aminosäuren
        (B) ART: Aminosäure
        (D) TOPOLOGIE: linear

    (ii) ART DES MOLEKÜLS: Protein
    (xi) SEQUENZBESCHREIBUNG: SEQ ID NO: 2:

Met Gly Val His Glu Cys Pro Ala Trp Leu Trp Leu Leu Leu Ser Leu
  1               5                  10                  15

Leu Ser Leu Pro Leu Gly Leu Pro Val Leu Gly Asp Tyr Lys Asp Asp
             20                  25                  30

Asp Asp Lys Ala Ala Ala Met Ser Gln Gly Ala Pro Ala Ser Val Leu
         35                  40                  45

Gly Ser Arg Pro Phe Asp Tyr Gly Leu Thr Trp Gln Thr Cys Ser Cys
     50                  55                  60

Arg Ala Asn Gly Ser Arg Phe Ser Thr Gly Glu Lys Val Trp Asp Arg
 65                  70                  75                  80

Gly Asn Val Thr Leu Gln Cys Asp Cys Pro Asn Gly Pro Trp Val Trp
                 85                  90                  95

Leu Pro Ala Phe Cys Gln Ala Ile Gly Trp Gly Asp Pro Ile Thr Tyr
            100                 105                 110

Trp Ser His Gly Gln Asn Gln Trp Pro Leu Ser Cys Pro Gln Tyr Val
        115                 120                 125

Tyr Gly Ser Ala Thr Val Thr Cys Val Trp Gly Ser Ala Ser Trp Tyr
    130                 135                 140

Ala Ser Thr Ser Gly Arg Asp Ser Lys Ile Asp Val Trp Ser Leu Val
145                 150                 155                 160

Pro Val Gly Ser Ala Thr Cys Thr Ile Ala Ala Leu Gly Ser Ser Asp
                165                 170                 175

Arg Asp Thr Val Pro Gly Leu Ser Glu Trp Gly Ile Pro Cys Val Thr
            180                 185                 190

Cys Val Leu Asp Arg Arg Pro Ala Ser Cys Gly Thr Cys Val Arg Asp
        195                 200                 205

Cys Trp Pro Glu Thr Gly Ser Val Arg Phe Pro Phe His Arg Cys Gly
    210                 215                 220

Val Gly Pro Arg Leu Thr Lys Asp Leu Glu Ala Val Pro Phe Val Asn
225                 230                 235                 240

Arg Thr Thr Pro Phe Thr Ile Arg Gly Pro Leu Gly Asn Gln Gly Arg
                245                 250                 255

Gly Asn Pro Val Arg Ser Pro Leu Gly Phe Gly Ser Tyr Ala Met Thr
            260                 265                 270

Arg Ile Arg Asp Thr Leu His Leu Val Glu Cys Pro Thr Pro Ala Ile
        275                 280                 285

Glu Pro Pro Thr Gly Thr Phe Gly Phe Phe Pro Gly Thr Pro Pro Leu
    290                 295                 300

Asn Asn Cys Met Leu Leu Gly Thr Glu Val Ser Glu Ala Leu Gly Gly
305                 310                 315                 320

Ala Gly Leu Thr Gly Gly Phe Tyr Glu Pro Leu Val Arg Arg Cys Ser
                325                 330                 335

Glu Leu Met Gly Ser Arg Asn Pro Val Cys Pro Gly Phe Ala Trp Leu
            340                 345                 350

Ser Ser Gly Arg Pro Asp Gly Phe Ile His Val Gln Gly His Leu Gln
        355                 360                 365

Glu Val Asp Ala Gly Asn Phe Ile Pro Pro Pro Arg Trp Leu Leu Leu
    370                 375                 380

Asp Phe Val Phe Val Leu Leu Tyr Leu Met Lys Leu Ala Glu Ala Arg
385                 390                 395                 400

Leu Val Pro Leu Ile Leu Leu Leu Leu Trp Trp Trp Val Asn Gln Leu
                405                 410                 415

Ala Val Leu Gly Leu Pro Ala Val Glu Ala
            420                 425

## Beschreibung

Die vorliegende Erfindung betrifft Antikörper gegen Hepatitis G-Viren und deren Fragmente. Die Erfindung betrifft außerdem ein Konjugat, welches diesen Antikörper oder ein Antikörperfragment gekoppelt an ein biologisches Molekül enthält. Schließlich betrifft die vorliegende Erfindung die Verwendung des Antikörpers zum diagnostischen Nachweis von Hepatitis G-Viren sowie Zellkulturen.

Neben Hepatitis A-Virus (HAV) und Hepatitis B-Virus (HBV), die schon seit langem bekannt sind, wurden in jüngerer Zeit weitere Hepatitis-assoziierte Viren charakterisiert, die verschiedenen Virusfamilien angehören. Sie verursachen eine Reihe zum Teil sehr schwerwiegender, aber durchaus unterschiedlicher Krankheiten, so daß eine möglichst frühzeitige und eindeutige Differentialdiagnose sehr wünschenswert ist. Die Hepatitis-Viren werden üblicherweise so benannt, daß ihnen ein fortlaufender Buchstabe des Alphabets zugeordnet wird. Alternativ dazu kann man neue Hepatitis-assoziierte Viren auch durch Ausschluß der bekannten Viren benennen. So wurde Hepatitis C-Virus (HCV) auch als NonA/NonB-Hepatitis-Virus bezeichnet (Choo et al., Science 244 (1989), 359-362). Die vorliegende Erfindung bezieht sich auf ein Virus, das in keine der von HAV-, HBV, HDV- und HEV vertretenen Virusfamilien eingeordnet werden kann. Die bislang über dieses neue Virus erhältlichen Informationen legen nahe, daß es wie HCV zur Familie der Flaviviridae gehört [Chambers et al., Annu. Rev. Microbiol. 44 (1990), 649-688). Aus diesen Daten geht jedoch auch hervor, daß es sich signifikant von HCV unterscheidet, und somit einer eigenen Virusgruppe angehört. Deshalb wird dieses neue Virus als Hepatitis G-Virus (HGV) bezeichnet (Linnen et al., Science 271 (1996), 505-508.

In W094/18217 ist ein Hepatitis-assoziiertes Virus beschrieben, das keiner der Gruppen HAV, HBV, HCV, HDV und HEV zugeordnet werden kann. Dieses Virus weist jedoch in seiner Nucleotidsequenz keinerlei Ähnlichkeit mit der in der vorliegenden Erfindung beschriebenen Sequenz auf.

In W095/21922 ist die Nucleinsäure- und Aminosäuresequenz von HGV beschrieben. In den Beispielen 13, 19 und 20 wird die rekombinante Expression von HGV-Polypeptiden in E.coli offenhart. Auf die in dieser Anmeldung offenbarten Sequenzen wird ausdrücklich Bezug genommen.

In WO95/32291 ist ebenfalls die Nucleinsäure- und Aminosäuresequenz von HGV beschrieben. In Beispiel 16 wird die rekombinante Expression von HGV in E.coli, in Insektenzellen und in Vaccinia beschrieben. Auf die in dieser Anmeldung offenbarten Sequenzen wird ausdrücklich Bezug genommen.

Virale Infektionen werden üblicherweise durch das Vorhandensein von Antigenen und/oder Antikörpern gegen diese Antigene in Körperflüssigkeiten, etwa dem Blutserum, nachgewiesen. Zum Nachweis der Antigene in einem Immunoassay werden Antikörper benötigt, welche spezifisch die viralen Antigene detektieren. Ebenso ist es für die Durchführung eines solchen immunologischen Tests erforderlich, die geeigneten Antikörper in ausreichender Menge bereitzustellen.

Aus der deutschen Patentanmeldung 196 13 406.4 sind neue Verfahren zur Herstellung von HGV-Antigenen und deren Verwendung zum diagnostischen Nachweis von HGV bekannt.

Dieser Stand der Technik stellt neue Verfahren zur Expression von HGV-Antigenen bereit. Als HGV-Antigene in diesem Sinne sind Polypeptide aus dem HGV-Genom geeignet, die mindestens eine antigene und/oder immunogene Determinante aufweisen. Aus dem HGV-Gesamtgenom sind die DNA-Sequenzbereiche bevorzugt, die für die putativen Hüllproteine E1 und E2 kodieren. Diese Hüllproteine sind zusammengesetzt aus einem aminoterminalen Hauptabschnitt, der an der Außenseite eines funktionellen Viruspartikels lokalisiert ist und eine entscheidende Rolle beim Andocken an den Wirtsorganismus spielt und einem kurzen carboxyterminalen hydrophoben Abschnitt, der in der Membran verankert ist.

Die o.g. Patentanmeldung offenbart weiterhin eine rekombinante Zelle, die an ihrer Oberfläche HGV-Antigene in membrangebundener Form präsentiert, insbesondere die Antigene E1 oder/und E2 oder immunologisch relevante Teilsequenzen davon.

Diese Zelle kann als diagnostisches Reagenz zum Nachweis von HGV, z.B. durch FACS-Analyse oder durch ELISA verwendet werden. Hierzu wird die Reaktion einer Zelle, die ein HGV-Antigen an ihrer Oberfläche präsentiert, mit einer Probeflüssigkeit, z.B. Humanserum, bestimmt. Bei Auftreten einer Reaktion ist davon auszugehen, daß in der getesteten Probe Anti-HGV-Antikörper enthalten sind.

Es bestehen jedoch noch keine Antikörper gegen die HGV-Epitope.

Klassischerweise werden Antikörper durch Immunisierung von Mäusen oder anderen Tieren mit Antigenen gewonnen. Monoklonale Antikörper mit dem Vorteil unbegrenzter Produktion und genau definierter Spezifität lassen sich dann aus dem zunächst polyklonalen Pool durch die Hybridom-Technologie selektieren (Köhler and Milstein,Nature 256 (1975), 495). Dabei werden Myelomzellen mit Milzzellen des immunisierten Tieres fusioniert.

Es war daher die Aufgabe der vorliegenden Erfindung, einen - vorzugsweise monoklonalen - Antikörper gegen ein HGV-Oberflächenantigen herzustellen, insbesondere gegen das Hüllprotein E2 von HGV. Es war eine weitere Aufgabe der Erfindung, diesen Antikörper als diagnostisches Reagenz zum Nachweis von HGV zu verwenden.

Durch die Erfindung werden monoklonale und polyklonale Antikörper gegen HGV-Oberflächenantigene zur Verfügung gestellt.

Es wird außerdem ein monoklonaler oder polyklonaler Antikörper gegen das HGV-E2 Oberflächenantigen zur Verfügung gestellt.

Als Immunogen zur Gewinnung der Antikörper sind Polypeptide und insbesondere Oberflächenantigene aus dem HGV-Genom (WO95/21922 und WO95/32291) oder Teilpeptidsequenzen davon, geeignet, die mindestens eine antigene oder/und immunogene Determinante aufweisen. Besonders bevorzugt ist das putative Hüllprotein E2 oder ein antigener oder/und immunogener Teilpeptidabschnitt davon, insbesondere ein Teilpeptidabschnitt aus dem aminoterminalen Bereich. Ebenfalls geeignet als Immunogen ist das putative Hüllprotein E1 oder ein antigener oder/und immunogener Teilpeptidabschnitt davon, der insbesondere aus dem aminoterminalen Bereich stammt. Immunisierungsverfahren unter Verwendung viraler Hüllproteine sind bekannt, vgl. HBsAg (Michel et al.,Bio/Technology 3 (1985), 561-566) und E2/NS1 von HCV (Lesniewski et al., J. Med. Virol. 45 (1995), 415-422).

Besonders bevorzugt werden die erfindungsgemäßen Antikörper jedoch durch ein mehrstufiges Immunisierungsverfahren hergestellt, welches eine DNA-Immunisierung (Davies et al., Ann.NY Acad.Sci. 772 (1995), 21-29) beinhaltet. Auf diese Weise gelingt es, die Oberlächenantigene dem tierischen Immunsystem möglichst authentisch, d.h. vorzugsweise in korrekter posttranslational prozessierter und gegebenenfalls glycosilierter und aus der Zelle exportierter Form, anzubieten. Im ersten Schritt des erfindungsgemäßen Immunisierungsverfahrens wird die für das Immunisierungsantigen kodierende DNA-Sequenz in einen eukaryontischen Expressionsvektor kloniert und dieses Konstrukt direkt in ein geeignetes Gewebe des Versuchstieres (z.B. Maus, Ratte, Kaninchen etc.) wie etwa den Skelettmuskel, injiziert. Auf dem Expressionsvektor befindet sich die für das Antigen kodierende DNA-Sequenz unter Kontrolle eines Promotors, von dem bekannt ist, daß er im jeweils verwendeten Gewebe des entsprechenden Versuchstieres aktiv ist.

Besonders bevorzugt wird eine für das Hüllprotein E2 oder einen Abschnitt davon kodierende DNA-Sequenz in einem Expressionsvektor, z.B. pcDNA3, im richtigen Leserahmen an die für ein aminoterminales Signalpeptid sowie gegebenenfalls für ein Markerepitop, z.B. das sogenannte FLAG-Epitop, kodierenden DNA-Sequenzen kloniert und zusammen mit den beiden Elementen als Fusionsprotein exprimiert. Die Signalsequenz kann z.B. die Erythropoietinsignalsequenz (Jacobs et al., Nature 313 (1985), 806-810) sein. Das FLAG-Epitop ist ein Oktapeptid (Hopp et al., Bio/Technology 6 (1988), 1204-1210), gegen das ein monoklonaler Antiköper kommerziell erhältlich ist, welcher zur Identifizierung und gegebenenfalls zur Aufreinigung des gewünschten Expressionsprodukts eingesetzt werden kann.

Bei Expression des Antigens im Gewebe des Versuchstieres findet die Proteinbiosynthese an den Ribosomen im Cytosol statt. Bei Expression der HGV-Antigene in operativer Verknüpfung mit aminoterminalen Signalsequenzen, z.B. 20 bis 30 Aminosäuren langen hydrophoben Sequenzen, die während der Proteinbiosynthese im Cytosol von sogenannten Signalerkennungsteilchen erkannt werden, werden die Ribosomen zum endoplasmatischen Reticulum (ER) dirigiert. Dort werden die entstehenden Polypeptidketten durch die ER-Membran geschleust, bis sie durch Stop-Transfersequenzen in der Membran arretiert werden. Im Lumen des ER werden die Proteine gegebenenfalls glycosiliert und anschließend im Golgi-Apparat weiter modifiziert. Schließlich werden sie für den Export in Richtung Plasmamembran sortiert. Auf diese Weise wird das Antigen dem Immunsystem in möglichst authentischer Form angeboten und kann zur Bildung von qualitativ hochwertigen Anti-HGV-Antikörpern führen.

Vorzugsweise beinhaltet das erfindungsgemäße Immunisierungsverfahren noch eine Auffrischungsimmunisierung, wobei dem Versuchstier eukaryontische Zellen injiziert werden, welche das entsprechende HGV-Oberflächenantigen membranständig exprimieren. Bezüglich der Herstellung solcher Zellen wird auf DE 196 13 406.6 verwiesen.

Aus dem immunisierten Versuchstier lassen sich nun polyklonale Antikörperzusammensetzungen oder monoklonale Antikörper gegen HGV-Oberflächenantigene gewinnen. Zur Herstellung einer polyklonalen, für HGV spezifischen Antikörperzusammensetzung findet vorzugsweise eine Reinigung des Serums statt, z.B. eine Affinitätschromatographie über eine mit dem entsprechenden Antigen, z.B. dem E2-Antigen beschichtete Säule.

Zur Gewinnung monoklonaler Antikörper wird auf die Hybridomtechnologie von Köhler und Milstein oder deren Weiterentwicklungen zurückgegriffen. Geeignete Antikörper-produzierende Hybridomzellen lassen sich beispielsweise durch Fusion von Milzzellen aus den immunisierten Tieren mit Myelomzellen in Anlehnung an Galfré and Milstein, Meth. Enzymol. 73 (1981), 3-46 erfolgen. Die Primärkulturen der Fusionszellen werden dann auf Synthese spezifischer Antikörper getestet. Spezifische Primärkulturen können mittels fluorescence activated cell sorting (FACS) in Mikrotiterplatten kloniert werden.

Somit betrifft die Erfindung einen polyklonalen oder monoklonalen Antikörper gegen ein Hepatitis G-Virus-Oberflächenantigen, der herstellbar ist durch ein Verfahren, das folgende Schritte umfaßt:
- Immunisierung von Versuchstieren mit einem Expressionsvektor, umfassend: einen Promotor, eine eukaryontische Signalsequenz, eine für das Hepatitis G-Virus-Oberflächenantigen kodierende DNA-Sequenz und gegebenenfalls eine Markersequenz,
- gegebenenfalls Auffrischungsimmunisierung der Versuchstiere mit Zellen, welche das HGV-Oberflächenantigen membranständig exprimieren und
- Gewinnung von für das Hepatitis G-Virus-Oberflächenantigen spezifischen polyklonalen oder monoklonalen Antikörpern aus dem immunisierten Versuchstier.

Der Antikörper, der gegen das HGV-E2-Oberflächenantigen zur Verfügung gestellt wird, richtet sich vorzugsweise gegen ein Polypeptid, welches kodiert ist von
(a) der zwischen Position 127 und 1290 von SEQ ID NO. 1 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nucleotidsequenz.

Es ist ein weiteres Charakteristikum des Antikörpers gegen das HGV-E2-Oberflächenantigen, daß er gegen ein Polypeptid gerichtet ist, das
(a) die zwischen Position 39 und 426 von SEQ ID NO. 2 angegebene Aminosäuresequenz oder
(b) eine mit der Sequenz aus (a) mindestens 80% homologe Aminosäuresequenz umfaßt.

Weiterhin werden Hybridomzellinien zur Verfügung gestellt, die erfindungsgemäße monoklonale Anti-E2-Antikörper produzieren. Diese Hybridomzellinien wurden gemäß den Bestimmungen des Budapester Vertrags bei der Deutschen Sammlung von Mikroorganismen und Zellkulturen GmbH (DSMZ), Mascheroder Weg 1b, D-38124 Braunschweig, hinterlegt. Die Hinterlegungsdaten sind wie folgt:
- Klon 11: DSM ACC 2280 19.09.1996
- Klon 17: DSM ACC 2284 24.09.1996
- Klon 30: DSM ACC 2285 24.09.1996

Die Erfindung betrifft auch einen aus den vorstehend angegebenen Zellinien gewinnbaren monoklonalen Antikörper sowie Antikörper mit äquivalenter Bindespezifität, welche vorzugsweise dasselbe Epitop wie die hinterlegten Antikörper erkennen.

Die Erfindung umfaßt außerdem jedes Fragment eines der oben beschriebenen Antikörper.

Noch ein weiterer Gegenstand der vorliegenden Erfindung ist ein Konjugat, welches einen der oben beschriebenen Antikörper oder ein entsprechendes Antikörperfragment enthält, das gekoppelt ist an ein biologisches Molekül.

In einer bevorzugten Ausführungsform umfaßt das biologische Molekül im Konjugat eine Markierungsgruppe. Als Markierungsgruppe kommen alle bekannten Markierungsgruppen in Frage, die in einem Testsystem nachgewiesen werden können, d.h. direkt oder indirekt nachweisbare Markierungsgruppen. Dabei ist unter einer direkt nachweisbaren Markierungsgruppe eine solche Gruppe zu verstehen, die ein direkt nachweisbares Signal erzeugt, z.B. eine radioaktive Gruppe, eine Enzymgruppe oder eine Lumineszenzgruppe. Besonders bevorzugt sind Enzymgruppen und Lumineszenzgruppen, insbesonders Elektrochemilumineszenzgruppen. Andererseits kann die Markierungsgruppe auch eine indirekt nachweisbare Gruppe sein, z. B. eine Biotin- oder Hapten-Gruppe, die durch Reaktion mit einem geeigneten Bindepartner (Streptavidin, Avidin bzw. Anti-Hapten-Antikörper), der wiederum eine signalerzeugende Gruppe trägt, nachweisbar ist. Hapten- oder Biotin-Gruppen können auch als Festphasenbindegruppen zur Immobilisierung der Antikörper an einer Festphase eingesetzt werden. Die Markierungs- bzw. Festphasenbindegruppe kann mit dem Antikörper auf bekannte Weise gekoppelt werden.

Ein weiterer Gegenstand der Erfindung betrifft die Verwendung der Antikörper als diagnostische Reagenzien zum Nachweis von HGV, z.B. durch FACS-Analyse oder durch ELISA. Hierzu wird die Reaktion der Antikörper mit einer Probeflüssigkeit, z.B. Humanserum, bestimmt. Bei Auftreten einer Reaktion ist davon auszugehen, daß in der getesteten Probe HGV-Antigene enthalten sind.

Zur Verwendung in einem diagnostischen Test werden die Antikörper vorzugsweise mit mindestens einer Markierungsgruppe oder Festphasenbindungsgruppe versehen, wie oben beschrieben.

Der Nachweis von HGV erfolgt insbesondere durch immunologische Bestimmung von HGV-Antigenen in einer Probeflüssigkeit, wobei man die Probeflüssigkeit mit mindestens einem erfindungsgemäßen Antikörper inkubiert und die Bindung nachweist. Dieses immunologische Bestimmungsverfahren kann an sich nach jedem bekannten Testformat erfolgen, z.B. in einem homogenen Immunoassay mit einer einzigen Reaktionsphase oder in einem heterologen Immunoassay mit mehr als einer Reaktionsphase. Vorzugsweise wird ein heterogenes Testformat verwendet, bei dem das Vorhandensein des Antigens in Anwesenheit einer Festphase nachgewiesen wird.

Eine Ausführungsform dieses Testformats ist der sog. Brückentest (siehe Beispiel 5). Dabei wird die Probeflüssigkeit mit mindestens zwei erfindungsgemäßen Antikörpern A1 und A2 inkubiert, wobei A1 an eine Festphase gebunden ist oder in einer an eine Festphase-bindefähigen Form vorliegt (sog. Fang-Antikörper) und A2 eine Markierungsgruppe trägt (sog. Nachweis-Antikörper). Das Antigen in der Probeflüssigkeit wird durch Bestimmung in der Markierung in der Festphase oder/und in der flüssigen Phase, vorzugsweise in der Festphase, über einen immobilisierten, d.h. an die Festphase gebundenen Immunkomplex nachgewiesen. Die Testdurchführung beinhaltet vorzugsweise ein Mischen der Probeflüssigkeit mit einem markierten A2 sowie mit einem festphasenseitigen A1, um einen markierten immobilisierten Komplex aus markiertem Antikörper, Antigen und festphasengebundenem Antikörper zu erhalten.

Ein weiteres Anwendungsgebiet der erfindungsgemäßen Antikörper ist die therapeutische Anwendung. Hierzu werden die erfindungsgemäßen Antikörper vorzugsweise in gereinigter Form hergestellt und dann in Form von injizierbaren Flüssigkeiten gebracht, wobei es sich um Lösungen oder Suspensionen handeln kann. Weitere Bestandteile sind z.B. Wasser, Salzlösungen, Glucose oder Glycerin. Die Antikörper können auch in Liposomen eingeschlossen sein. Die Antikörper werden üblicherweise parenteral durch Injektion, vorzugsweise subkutan oder intramuskulär appliziert.

Weiterhin wird die vorliegende Erfindung durch die nachfolgenden Beispiele, Sequenzprotokolle und Abbildungen beschrieben. Es zeigen:
- SEQ ID NO. 1:: eine DNA-Nucleotidsequenz, die für HGV-E2 kodiert plus aminoterminalem Fusionsanteil
- SEQ ID NO. 2:: eine Aminosäuresequenz des HGV-E2 plus aminoterminalem Fusionsanteil
- Fig. 1:: Färbung von FLAG-E2 exprimierenden CHO Zellen mit Anti-FLAG-M1 und Anti-E2 MAKs 3, 5, 6, 11, 13, 17, 19, 30
- Fig. 2:: Färbung von CHO-Zellen (ausgefüllter Bereich) und FLAG-E2 exprimierenden CHO-Zellen (offener Bereich) mit den Anti-E2-MAKs 3, 5, 6, 11, 13, 17, 19, 30.

### BEISPIELE

### Beispiel 1

### Klonierung des Expressionskonstruktes HGV-E2

Zur Manipulation der DNA wurden Standardmethoden benutzt, wie sie bei Sambrook et al. (1989) in Molecular cloning: A Laboratory Manual, Cold Spring Harbor Laboratory, New York und Ausubel et al. (1989) in Current Protocols in Molecular Biology, John Wiley & Sons, New York beschrieben sind.

Die Manipulation von DNA wurde im E. coli K12-Stamm DH5α durchgeführt.

Als Expressionsvektor zur DNA-Immunisierung wurde ein Derivat des Vektors pcDNA3 mit CMV-Promotor und BGH poly-Adenylierungssignal (Invitrogen BV, NV Leek, Niederlande) verwendet. Hierzu wurde der Vektor pcDNA3 durch den Austausch des Neomycin-Resistenzgens gegen ein Dehydrofolatreduktase (DHFR)-Gen modifiziert. Dies erfolgte durch Restriktionsspaltung von pcDNA3 mit AvrII/Bst1107I, Isolierung des ca. 4 kBp langen Vektorfragments und Insertion eines ca. 700 Bp langen AvrII/Bst1107I DHFR-Fragments [Setzer et al. (1982) J. Biol. Chem. 257, 5143 - 5147; Crouse et al. (1982) J. Biol. Chem. 257, 7887 - 7897].

Die für das Hüllprotein E2 kodierende DNA-Sequenz wurde in den resultierenden Vektor pcDNA3-DHFR in operativer Verknüpfung an eine Signalpeptidsequenz sowie an eine für ein sog. FLAG-Epitop kodierende DNA-Sequenz kloniert. Als Signalsequenz wurde die Erythropoietinsignalsequenz [Jacobs et al. (1985) Nature 313, 806 - 810] als 93 Bp langes EcoRI/EheI- Fragment (Position 1 bis 93 von SEQ.ID.No. 1) eingesetzt. Das FLAG-Epitop ist ein kurzes Achterpeptid [Hopp et al. (1988) Bio/Technology 6, 1204 - 1210], gegen das ein monoklonaler Antikörper Anti-Flag-M1, Kodak Eastman) erhältlich ist, welcher zur Aufreinigung und Identifizierung des gewünschten Expressionsproduktes eingesetzt werden kann. Zur Darstellung der für das FLAG-Epitop kodierenden DNA-Sequenz wurden zwei Oligonukleotide miteinander hybridisiert und nach Kinasierung als Linker eingesetzt (entsprechend Position 94 - 126 von SEQ ID NO. 1).

Zur Herstellung des E2-Expressionsvektors wurde das Plasmid pcDNA3-DHFR mit EcoRI und NotI verdaut, das ca. 5,9 kBp lange Vektorfragment isoliert und in einer 3-Wege-Ligation mit dem EcoRI/EheI- Signalsequenzfragment und dem FLAG-Linker ligiert.

Die verwendete HGV-cDNA stammte von Genelabs Technologies Inc., Redwood City, CA, USA (WO 95/32291). Das fast vollständige HGV Genom lag als ca. 9,3 kBp langes XbaI/EcoRI-Fragment vor, welches in die entsprechenden Restriktionsstellen des Vektors pGEM-3Z (Promega Corp., Madison, WI, USA) kloniert worden war. Die für E2 kodierenden DNA- Sequenzen (Position 127 - 1290 von SEQ.ID.NO. 1) wurden mit geeigneten Oligonukleotiden mittels PCR amplifiziert und als NotI/XbaI-Fragmente in frame an die für Signalpeptid und FLAG-Epitop kodierenden DNA-Sequenzen kloniert.

### Beispiel 2

### Herstellung monoklonaler Antikörper gegen HGV-E2

### 2.1. DNA-Immunisierung

Für die Immunisierung wurden weibliche BALB/c-Mäuse verwendet. Zur Steigerung der Effizienz der DNA-Aufnahme, die im regenerierenden Muskel etwa zehnfach höher sein soll als im unbehandelten Muskel, wurden die Tiere vor der ersten Immunisierung mit dem Schlangengift Latoxan (Rosans, Frankreich) behandelt. Dazu wurden jeweils 80 µl einer 10 µM Lösung des Gifts in beide Schienbein-Muskel der Maus injiziert. Fünf Tage später wurde mit der Immunisierung begonnen, jeder Maus wurde fünfmal (Woche 0, 5, 10, 11, 12) Plasmid-DNA gespritzt. Dabei wurden 50 µg DNA in jeden Schienbeinmuskel injiziert, d.h. 100 µg pro Tier und Immunisierung. Die Aufreinigung der Plasmid-DNA erfolgte über Qiagen-Säulen gemäß den Herstellerangaben.

Vor der Milzentnahme in Woche 20 wurden die Tiere geboostet. Dazu wurden pro Tier 10⁷ CHO-Zellen, die das HGV-E2 membranständig exprimieren, intravenös injiziert.

### 2.2. Fusion und Klonierung

Die Fusion von Milzzellen der immunisierten Mäuse mit Myelomzellen erfolgte in Anlehnung an Galfré and Milstein (1981) Meth. Enzymol. 73, 3-46. Dabei wurden ca. 10⁸ Milzzellen der immunisierten Maus mit 2 x 10⁷ Myelomzellen (P3X63-Ag8-653, ATCC CRL1580) gemischt und abzentrifugiert. Die Zellen wurden dann einmal in RPMI 1640-Medium w/o FKS gewaschen und bei 400 g erneut abzentrifugiert. Der Überstand wurde abgekippt, das Zellsediment durch Klopfen leicht aufgelockert, 1 ml PEG (Molekulargewicht 4000, Life Technologies, Kat. Nr. 14030035) innerhalb einer Minute dazugegeben und durch vorsichtiges Schwenken in einem 37°C warmen Wasserbad mit den Zellen gemischt. Anschließend wurden 5 ml RPMI 1640-Medium w/o FKS innerhalb von 5 min. zugetropft und durch dauerndes Schwenken in einem 37°C warmen Wasserbad gemischt. Nach der Zugabe von 25 ml RPMI 1640 Medium w/o FKS wurden die Zellen 10 min bei 400 g abzentrifugiert. Das Zellpellet wurde in RPMI 1640-Medium, 10 % FKS aufgenommen und in Hypoxanthin-Azaserin Selektionsmedium (lOO mmol/l Hypoxanthin, 1 µg/ml Azaserin in RPMI 1640, 10 % FKS) eingesät. Als Wachstumsfaktor wurde dem Medium Interleukin-6 (Boehringer Mannheim, Kat.Nr. 1444 581) zugegeben. Nach ca. 10 Tagen wurden die Primärkulturen auf Synthese E2-spezifischer Antikörper getestet (siehe Beispiel 3). E2-spezifische Primärkulturen wurden mittels fluorescence activated cell sorting (FACS) in Mikrotiterplatten kloniert. Hierbei wurde dem Medium als Wachstumszusatz Interleukin-6 zugegeben. Die Reinigung der Antikörper aus Ascites-Flüssigkeit von Mäusen und die Derivatisierung mit Biotin oder Digoxigenin erfolgte gemäß proteinchemisch üblichen Methoden.

### Beispiel 3

### Bestimmung der Spezifität der produzierten Antikörper

Um die Spezifität der Antikörper im Kulturüberstand der Hybridomzellen zu bestimmen, wurde in zwei parallelen ELISA-Ansätzen die Reaktivität mit CHO-Zellen bestimmt, die membranständig entweder das FLAG-E2-Fusionsprotein oder den ebenfalls mit einer NH₂-terminalen FLAG-Sequenz versehenen humanen Urokinase-Rezeptor exprimieren. Dazu wurden die Zellen am Vortag des Versuchs in einer Dichte von ca. 4 x 10⁴ pro well einer Mikrotiterplatte ausgesät. Am nächsten Tag wurden zunächst unspezifische Bindungsstellen durch zweistündige Inkubation mit 200 µl RPMI 1640- Medium, 10 % FKS, 1 % BycoC pro well blockiert. Anschließend wurden 100 µl der Zellkulturüberstände in das jeweilige well pipettiert und 60 min. bei Raumtemperatur inkubiert. Nach dreimaligem Waschen mit Dulbecco's PBS, 0,02 % Tween 20, wurde Anti-Maus IgG-Peroxidase-Fab-Fragment (Boehringer Mannheim Kat.Nr. 1500 686), in einem Volumen von 100 µl und einer Konzentration von 50 mU/ml zu den Zellen gegeben. Nach 60 min. Inkubation bei Raumtemperatur und dreimaligem Waschen mit Dulbecco's PBS, 0,02 % Tween 20 wurde ABTS^{R} als Substrat eingesetzt und die Farbänderung nach 30 - 60 min. im ELISA-Reader bei 405/490 nm gemessen.

Insgesamt wurden 8 Hybridom-Klone identifiziert (Anti-HGV-E2 Klone 3, 5, 6, 11, 13, 17, 19, 30), deren monoklonale Antikörper spezifisch EGV-B2 exprimierende CHO-Zellen erkennen (MAKs 3, 5, 6, 11, 13, 17, 19, 30).

### Beispiel 4

### FACS-Analytik mit HGV-E2 exprimierenden CHO-Zellen

Bei diesem Verfahren wurden CHO-zellen, die das FLAG-E2-Fusionsprotein membranständig exprimieren, nacheinander mit einem der E2-spezifischen MAKs und dem Anti-FLAG-Ml gefärbt. Da beide Epitope auf einem Molekül lokalisiert sind, erlaubt dieser Versuch eine Aussage über mögliche Epitopüberlappungen und somit über den Einfluß des FLAG-Epitops auf die Bindung von E2-spezifischen Antikörpern.

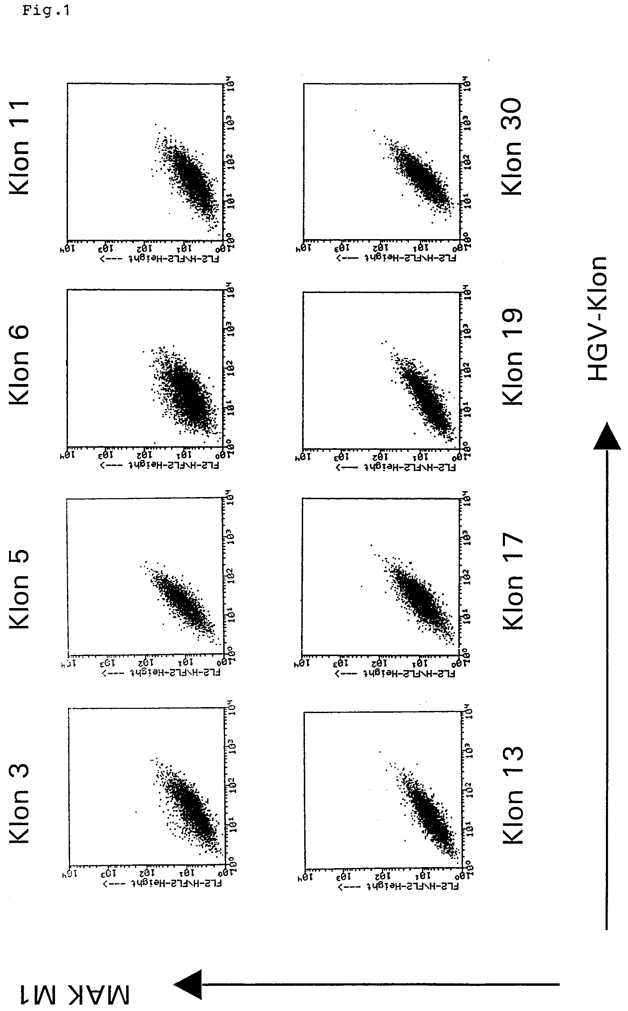

Die Zellen wurden von dem Kulturgefäß mit 0,02 % EDTA in PBS abgelöst und in PBS gewaschen. Je 2 x 10⁵ Zellen wurden in 100 µl Dulbecco's PBS, 0,2 % Rinderserumalbumin, 0,02 % NaN₃ resuspendiert und mit den MAKs 3, 5, 6, 11, 13, 17, 19 oder 30 (je 2 µg/ml) für 15 min. auf Eis inkubiert, mit dem gleichen Puffer zweimal gewaschen und für weitere 15 min mit Anti-Maus Ig-Fluorescein-(Fab')₂-Fragment (Boehringer Mannheim, Kat. Nr. 1295 750), inkubiert. Nach zweimaligem Waschen wurden die Zellen zur Blockierung freier Anti-Maus-IgG Bindestellen mit Maus-IgG (Sigma) in einer Konzentration von 10 µg/ml für 15 min. auf Eis inkubiert. Anschließend wurde biotinylierter Anti-FLAG-M1 in einer Konzentration von 0,35 µg/ml zum Ansatz gegeben. Nach 15 min. Inkubation auf Eis wurde zweimal gewaschen und der zweite, diesmal mit Streptavidin-R-Phycoerythrin (Boehringer Mannheim, Kat. Nr. 1428 560) fluoreszenzmarkierte MAK für 15 min. auf Eis zugegeben. Nach zweimaligem Waschen wurden die Zellen im Durchflußzytometer analysiert (Fig. 1). Die Doppelfärbung ergab jeweils eine Gerade, die in unterschiedlicher Neigung vorliegt. Eine Steigung von 45°, wie sie bei den MAKs 5, 17 und 30 vorliegt, ist typisch für die Erkennung zweier, voneinander unabhängiger Epitope. Eine von 45° abweichende Steigung wie im Falle der MAKs 3, 11, 13 und 19 könnte auf eine gegenseitige sterische Behinderung bei der Bindung hinweisen.

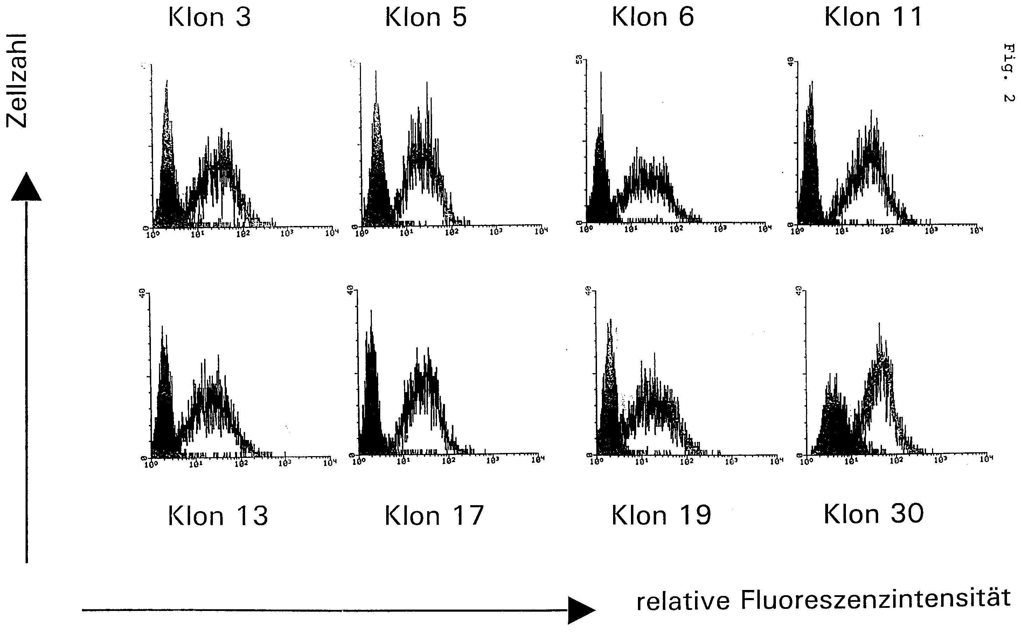

Um eine unspezifische Bindung der Anti-E2-MAKs an CHO-Zellen auszuschließen, wurden zusätzlich CHO-Zellen als Negativ-Referenz gefärbt. Die Färbung mit Anti-FLAG-M1 wurde in diesem Fall weggelassen (Fig. 2). Dabei zeigte nur MAK 30 eine leicht unspezifische Reaktion mit CHO-Zellen, während bei den anderen sieben MAKs keine Hintergrundfärbung detektierbar war.

### Beispiel 5

### Untersuchung der Epitopüberlappung

Der Test wurde als Brückentest durchqeführt, dabei wurde isoliertes HGV-E2 als Antigen zur Charakterisierung der E2-spezifischen MAKs benutzt. Das Antigen wurde dabei über einen biotinylierten, E2-spezifischen Fang-Antikörper gebunden, welcher seinerseits an eine Streptavidin-beschichtete ELISA-Platte gekoppelt war.

### a) Vorbereitunq der ELISA-Platten:

Streptavidin-beschichtete ELISA-Mikrotiterplatten (Microcode, Streptavidin MTP F8) wurden 60 - 120 min mit 50 µl biotinyliertem MAK (2 µg/ml in Dulbecco's PBS, 0,2 % Rinderserumalbumin) inkubiert und anschließend dreimal mit 0,9 % NaCl, 0,05 % Tween 20 gewaschen.

### b) Zellyse:

Die HGV-E2 exprimierenden Zellen wurden von dem Kulturgefäß in 0,02 % EDTA in PBS abgelöst und in PBS gewaschen. Anschließend wurden die Zellen in PBS, 0,5 % Nonidet P 40, Protease-Mix (Boehringer Mannheim, Kat. Nr. 1206 893) lysiert. Dazu wurden je 10⁷ Zellen/ml Solubilisierungslösung für zwei Stunden auf Eis inkubiert. Ungelöstes Material wurde durch Zentrifugation abgetrennt.

### c) Kopplung der Antigene und Assay:

Mit dem Zellysat (verdünnt in PBS, 0,1 % Nonidet P40) wurden die nach a) präparierten ELISA-Platten 60 min. bei Raumtemperatur inkubiert. Nach dreimaligem Waschen mit PBS, 0,1 % Nonidet P40 wurde der zweite, diesmal Digoxigenin-markierte MAK zugegeben und 90 min. bei Raumtemperatur inkubiert. Nach mehrmaligem Waschen mit PBS, 0,1 % Nonidet P40 wurde anschließend eine Stunde mit anti-Digoxigenin IgG-POD inkubiert. Nach intensivem Waschen mit PBS, 0,1 % Nonidet P40 wurde ABTS^{R} als Substrat eingesetzt und die Farbänderung nach 30 - 60 min im ELISA-Reader bei 405/490 nm gemessen.

Die MAKs 3, 5, 11, 13, 17, 19, 30 wurden in allen möglichen Kombinationen als biotinylierte Fang-Antikörper und als Digoxigenin-markierte Nachweis-Antikörper eingesetzt. Da im oben beschriebenen ELISA-Format alle Reaktionsschritte sequenziell verlaufen, ist für einen guten Fang-Antikörper eine niedrige Dissoziationskonstante essentiell. Diese Voraussetzung erfüllten die MAKs 5, 11, 17 und 30. Demgegenüber ließen sich die MAKs 3, 13 und 19 nur als Nachweis-Antikörper einsetzen. Die gleichzeitige Verwendung eines MAK als Fang- und Nachweis-Antikörper war bei diesem Test nicht möglich, was auf eine monovalente Konformation des Antigens hinweist.

Der Test ermöglicht auch die Untersuchung einer möglichen Epitopüberlappung der einzelnen MAKs. Offensichtlich erkennen die MAKs 5 und 17 dasselbe Epitop, da diese Kombination im Brückentest zu keinem Signal führte. Demgegenüber kompetitierten die MAKs 5 und 17 nicht mit den MAKs 11 oder 30. Dies zeigt, daß von diesen MAKs mindestens drei verschiedene Epitope auf HGV-E2 erkannt werden.

## Patentansprüche

1. Monoklonaler Antikörper gegen ein Hepatitis G-Virus Oberflächenantigen.

2. Polyklonale Antikörperzusammensetzung gegen Hepatitis G-Virus Oberflächtenantigene.

3. Monoklonaler Antikörper gegen das Hepatitis G-Virus-E2-Oberflächenantigen.

4. Polyklonale Antikörperzusammensetzung gegen das Hepatitis G-Virus-E2-Oberflächenantigen.

5. Antikörper nach Anspruch 3 oder 4,
**dadurch gekennzeichnet,**
daß er gegen ein Polypeptid gerichtet ist, welches kodiert ist von
(a) der zwischen Position 127 und 1290 von SEQ ID NO. 1 angegebenen Nucleotidsequenz,
(b) einer der Sequenz aus (a) im Rahmen der Degeneration des genetischen Codes entsprechenden Sequenz oder/und
(c) einer mit den Sequenzen aus (a) oder/und (b) unter stringenten Bedingungen hybridisierenden Nucleotidsequenz.

6. Antikörper nach einem der Ansprüche 3, 4 oder 5,
**dadurch gekennzeichnet,**
daß er gegen ein Polypeptid gerichtet ist, das
(a) die zwischen Position 39 und 426 von SEQ ID NO. 2 angegebene Aminosäuresequenz oder
(b) eine mit der Sequenz aus (a) mindestens 80% homologe Aminosäuresequenz umfaßt.

7. Polyklonaler oder monoklonaler Antikörper gegen ein Hepatitis G-Virus-Oberflächenantigen, der herstellbar ist durch ein Verfahren, das folgende Schritte umfaßt:
- Immunisierung von Versuchstieren mit einem Expressionsvektor, umfassend: einen Promotor, eine eukaryontische Signalsequenz, eine für das Hepatitis G-Virus-Oberflächenantigen kodierende DNA-Sequenz und gegebenenfalls eine Markersequenz,
- gegebenenfalls Auffrischungsimmunisierung der Versuchstiere mit Zellen, welche das HGV-Oberflächenantigen membranständig exprimieren und
- Gewinnung von für das Hepatitis G-Virus-Oberflächenantigen spezifischen polyklonalen oder monoklonalen Antikörpern aus dem immunisierten Versuchstier.

8. Antikörper nach Anspruch 3, 5, 6 oder 7, der aus der Zellinie "11" (DSM ACC 2280) gewinnbar ist oder ein Antikörper mit äquivalenter Bindespezifität.

9. Antikörper nach Anspruch 3, 5, 6 oder 7, der aus der Zellinie "17" (DSM ACC 2284) gewinnbar ist oder ein Antikörper mit äquivalenter Bindespezifität.

10. Antikörper nach Anspruch 3, 5, 6 oder 7, der aus der Zellinie "30" (DSM ACC 2285) gewinnbar ist oder ein Antikörper mit äquivalenter Bindespezifität.

11. Konjugat, welches einen Antikörper nach einem der Ansprüche 1 bis 10 oder ein Fragment eines solchen Antikörpers gekoppelt an ein biologisches Molekül, enthält.

12. Verwendung eines der Antikörper oder Konjugate nach einem der Ansprüche 1 bis 11 als Diagnostikreagenz zum Nachweis von HGV.

13. Zellkultur mit der Hinterlegungsnummer DSM ACC2280.

14. Zellkultur mit der Hinterlegungsnummer DSM ACC2284.

15. Zellkultur mit der Hinterlegungsnummer DSM ACC2285.
